# EUROPÄISCHE PATENTSCHRIFT

(11) **EP 1 742 880 B1**
(45) Veröffentlichungstag und Bekanntmachung des Hinweises auf die Patenterteilung: **28.05.2008**
(21) Anmeldenummer: 05731904.8
(22) Anmeldetag: 14.04.2005
(51) Int. Cl.: C02F 1/26, C07C 37/72

(54) **EXTRAKTION PHENOLHALTIGER ABWASSERSTRÖME**
EXTRACTION OF PHENOLIC WASTE WATER FLOWS
EXTRACTION DE COURANTS D'EAUX USEES PHENOLIQUES

(30) Priorität: 24.04.2004 DE 102004020113
(43) Veröffentlichungstag der Anmeldung: 17.01.2007
(73) Patentinhaber: Bayer MaterialScience AG, 51368 Leverkusen (DE)
(72) Erfinder: BLASCHKE, Ulrich, 47829 Düsseldorf (DE); WESTERNACHER, Stefan, 47906 Kempen (DE)
(86) Internationale Anmeldenummer: PCT/EP2005/003923
(87) Internationale Veröffentlichungsnummer: WO 2005/102936

(56) Entgegenhaltungen:
- EP-A- 0 758 636
- US-A- 4 374 283
- PATENT ABSTRACTS OF JAPAN Bd. 2000, Nr. 03, 30. März 2000 (2000-03-30) & JP 11 347536 A (TEIJIN LTD), 21. Dezember 1999 (1999-12-21)
- SCHAEFER H G: "SAUBERES ABWASSER AUS DER PHENOLHARZPRODUKTION" WASSER, LUFT UND BODEN, VEREINIGTE FACHVERLAG KRAUSSKOPF- INGENIEUR DIGEST, MAINZ, DE, Bd. 35, Nr. 6, 1. Juni 1991 (1991-06-01), Seiten 28-29, XP000215785 ISSN: 0938-8303

## Beschreibung

Die vorliegende Anmeldung betrifft ein Verfahren zur Extraktion phenolhaltiger Abwasserströme.

In industriellen Prozessen, wie beispielsweise bei der Phenolherstellung nach dem Cumol-Prozess oder der Herstellung von Bisphenol A durch Kondensation von Phenol und Aceton fallen phenolhaltige, wässrige Lösungen als Abwasserströme an.

Aufgrund der Löslichkeit von Phenol in Wasser (oberhalb von 65,3°C sind beide unbegrenzt miteinander mischbar, bei Raumtemperatur lösen sich noch fast 10 Gew.% Phenol in Wasser) und der Bildung von Azeotropen bei der Destillation solcher Lösungen ist die Abtrennung des Phenols aus solchen Abwasserströmen nicht durch eine einfache Destillation möglich. (J. M. Sørensen, W. Arlt, Liquid-Liquid Equilibrium Data Collection, Chemistry Data Series V/1, S. 356 - 361, Dechema, Frankfurt 1979; Ullmann's Encyclopedia of Industrial Chemistry, Vol. A 19, S. 299 - 312,5. Edition, VCH Weinheim 1991).

Bei der Herstellung von Bisphenol A mit Hilfe von sauren Ionenaustauschern muss das in der Reaktion gebildete Wasser entfernt werden, da sonst die Aktivität des Katalysators verringert wird. Dies geschieht zunächst durch eine destillative Abtrennung des Reaktionswassers vom Bisphenol A, seinen Isomeren und dem im Überschuss im Bisphenol A-Prozess als Lösungsmittel eingesetzten Phenol. Dieses so destillativ abgetrennte Wasser ist stark phenolhaltig (1-15 Gew.% Phenol) und ggf. auch acetonhaltig (0-5 Gew.%). Neben diesen beiden Hauptverunreinigungen können auch andere organische Komponenten wie Methanol, Aceton-Eigenkondensationsprodukte wie beispielsweise Mesityloxid, der im Bisphenol A-Prozess eingesetzte schwefelhaltige Cokatalysator (ein Thiol) sowie Abbau- und Folgeprodukte dieser Verbindungen in dem abgetrennten Wasser enthalten sein. Aber auch andere, in einem solchen Prozess anfallende wasserhaltige Ströme können - neben weiteren organischen Komponenten - Phenol enthalten (z.B. Wasser für Gleitringdichtungen oder Vakuumanlagen).

Aufgrund der Giftigkeit von Phenol und aus ökonomischen Gründen (Rückgewinnung von Phenol) werden daher die phenolhaltigen Abwässer in einer Extraktion mit einem organischen Lösungsmittel aufgereinigt. Hierbei kommen beispielsweise Benzol, Cumol, Diisopropylether oder Methylisobutylketon (MIBK) zum Einsatz (vgl. z.B. EP 0 758 636 A). Die Extraktion kann nach dem Fachmann bekannten Techniken (Kastenextrakteur, Mixer-Settler Systeme, Gegenstromextraktion etc.) durchgeführt werden. In einem anschließenden Schritt wird das so behandelte Abwasser meist noch über eine Dampfstripperkolonne aufgearbeitet, um den Restgehalt an Extraktionsmittel zu reduzieren. Dies ist wichtig, da Restgehalte von Extraktionsmitteln zu einer hohen organischen Belastung des Abwassers führen. Durch eine Reduzierung des Gehalts an Extraktionsmittel im Abwasser kann der Einsatz von Dampf und somit Energiekosten eingespart werden. Das so behandelte Abwasser wird dann einer biologischen Abwasserbehandlung in einer Kläranlage unterworfen.

Es wurde nun gefunden, dass bei der Extraktion phenolhaltiger, wässriger Lösungen mit MIBK als Extraktionsmittel der Restgehalt an MIBK nach der Extraktion verringert werden kann, wenn das für die Extraktion eingesetzte MIBK zusätzlich anteilig Anisol und gegebenenfalls Mesitylen enthält.

Die Erfindung betrifft ein Verfahren zur Extraktion phenolhaltiger wässriger Lösungen, bei dem als Extraktionsmittel ein Gemisch enthaltend 60 bis 99 Gew.-% Methylisobutylketon, 1 bis 40 Gew.-% Anisol und 0 bis 20 Gew.-% Mesitylen, bezogen auf das Gemisch, eingesetzt wird.

Bevorzugt ist dabei ein Verfahren zur Extraktion phenolhaltiger wässriger Lösungen, bei dem als Extraktionsmittel ein Gemisch enthaltend 75 bis 95 Gew.-% Methylisobutylketon, 5 bis 25 Gew.-% Anisol und 0 bis 10 Gew.-% Mesitylen, bezogen auf das Gemisch, eingesetzt wird.

Durch die Extraktion eines phenol- und acetonhaltigen Abwassers mit einem Gemisch aus MIBK und Anisol anstelle von reinem MIBK wird der Restgehalt an MIBK im Abwasser unter gleichen Extraktionsbedingungen deutlich verringert. Dabei bleibt der Extraktionsgrad für Phenol und Aceton praktisch gleich.

Zwar finden sich bei Verwendung eines Gemisches von MIBK und Anisol geringe Mengen Anisol im Abwasser wieder, allerdings ist die Abnahme des MIBK-Gehaltes deutlich größer als die Zunahme des Gehaltes an Anisol, so dass insgesamt ein Abwasser mit geringeren Gehalten an organischen Bestandteilen resultiert. Durch den Zusatz von Mesitylen zu dem Extraktionsgemisch kann zudem der Gehalt an Anisol im Wasser nach der Extraktion wieder verringert werden bei gleichbleibendem Extraktionseffekt und Restgehalt an MIBK.

Der Phenolgehalt in der wässrigen Lösung (dem Abwasser) beträgt bevorzugt 1 bis 15 Gew.-%, bezogen auf die wässrige Lösung.

Darüber hinaus können bis zu 5 Gew.% Aceton, bezogen auf die wässrige Lösung, enthalten sein.

Zur Verbesserung der Extraktion kann die wässrige Lösung zusätzlich Natriumsulfat enthalten und/oder die wässrige Lösung z.B. mittels Schwefelsäure oder anderen Säuren auf pH-Werte <7 angesäuert werden.

Die Extraktion kann nach dem Fachmann bekannten Techniken (Mehrstufenextraktion, Kastenextrakteur, Mixer-Settler Systeme, Gegenstromextraktion etc.) durchgeführt werden.

Nach der Extraktion kann das Abwasser zusätzlich in einer Kolonne ggf. unter Dampfeinspeisung destillativ von Resten des Extraktionsmittels befreit werden.

Die nach der Destillation oder Extraktion anfallende wässrige Phase kann zusätzlich einer Behandlung in einer biologischen Kläranlage zugeführt werden.

Die phenolhaltige, wässrige Lösung kann außerdem vor der Extraktion einer Destillation unterworfen werden, bei der beispielsweise enthaltene Ketone oder andere organische Komponenten ganz oder teilweise abgetrennt werden.

Das aus der phenolhaltigen, wässrigen Lösung extrahierte Phenol sowie gegebenenfalls andere extrahierte organische Komponenten (insbesondere gegebenenfalls extrahiertes Aceton) können destillativ von dem Extraktionsmittel getrennt und so wiedergewonnen werden. Das so aufgereinigte Extraktionsmittel kann erneut für die Extraktion verwendet werden.

Die nach Extraktion bzw. nach Destillation anfallende wässrige Phase kann vor einer Behandlung in einer biologischen Kläranlage zusätzlich mit Aktivkohle aufgereinigt werden.

Das erfindungsgemäße Verfahren wird bevorzugt bei der Aufreinigung von Abwässern aus der Herstellung von Bisphenol A eingesetzt. Bei dem Verfahren zur Herstellung von Bisphenol A können beispielsweise Phenol und Aceton an sulfonsauren Kationenaustauschern als Katalysator gegebenenfalls in Gegenwart eines Thiols als Cokatalysator umgesetzt werden, das erhaltene Bisphenol A ganz oder teilweise in Form eines Bisphenol A-Phenol-Adduktkristalles auskristallisiert und abfiltriert und anschließend das Filtrat destillativ ganz oder teilweise vom Wasser befreit werden. Ebenso kann es bei einem Verfahren zur Herstellung von Bisphenol A zur Anwendung kommen, bei dem die Abtrennung des Reaktionswassers abweichend von dem oben beschriebenen Verfahren ganz oder teilweise vor der Kristallisation der Bisphenol A-Phenol-Adduktkristalle erfolgt. Darüber hinaus ist es geeignet für die Extraktion von Abwässern aus Verfahren zur Herstellung von Bisphenol A, bei denen während oder vor dem Kristallisationsschritt Wasser (gegebenenfalls in einer Mischung mit Phenol oder mit anderen organischen Lösungsmitteln) hinzugegeben wird.

### Beispiele:

### Beispiel 1 (Vergleichsbeispiel)

In einem 500 ml Schütteltrichter werden 170 ml einer phenolhaltigen, wässrigen Lösung (Prozessabwasser aus der Herstellung von Bisphenol A) 15 Sekunden lang mit 100 ml MIBK geschüttelt. Man lässt anschließend den Schütteltrichter für 5 Minuten stehen, lässt dann die wässrige Phase ab und untersucht diese per Gaschromatographie. Ergebnisse siehe Tabelle 1.

### Beispiel 2 (erfindungsgemäß)

Beispiel 2 wird analog zu Beispiel 1 durchgeführt mit dem Unterschied, dass anstelle von 100 ml MIBK ein Gemisch aus 80 ml MIBK und 20 ml Anisol verwendet wird. Ergebnisse siehe Tabelle 1.

### Beispiel 3 (erfindungsgemäß)

Beispiel 3 wird analog zu Beispiel 1 durchgeführt mit dem Unterschied, dass anstelle von 100 ml MIBK ein Gemisch aus 83 ml MIBK, 12 ml Anisol und 5 ml Mesitylen verwendet wird. Ergebnisse siehe Tabelle 1.

**Tabelle 1: Ergebnisse der Extraktionsversuche [Gew.% in der wässrigen Phase nach Extraktion]**

| **Verbindung** | **Beispiel 1** | **Beispiel 2** | **Beispiel 3** |
|---|---|---|---|
| Phenol | 0,21 | 0,23 | 0,25 |
| MIBK | 1,40 | 1,00 | 1,05 |
| Aceton | 0,017 | 0,015 | 0,017 |
| Anisol | < 0,005 | 0,044 | 0,025 |
| Mesitylen | < 0,005 | < 0,005 | < 0,005 |
| Summe | ca. 1,63 | ca. 1,29 | ca. 1,35 |

## Patentansprüche

1. Verfahren zur Extraktion phenolhaltiger wässriger Lösungen, bei dem als Extraktionsmittel ein Gemisch enthaltend 60 bis 99 Gew.-% Methylisobutylketon, 1 bis 40 Gew.-% Anisol und 0 bis 20 Gew.-% Mesitylen, bezogen auf das Gemisch, eingesetzt wird.

2. Verfahren nach Anspruch 1, bei dem der Phenolgehalt in den zu extrahierenden phenolhaltigen wässrigen Lösungen 1-15 Gew.% beträgt.

3. Verfahren nach einem der Ansprüche 1 oder 2, bei dem die phenolhaltigen, wässrigen Lösungen ganz oder zum Teil aus der Produktion von Bisphenol A stammen.

4. Verfahren nach einem der Ansprüche 1 bis 3, bei dem die phenolhaltigen, wässrigen Lösungen Natriumsulfat enthalten.

5. Verfahren nach einem der Ansprüche 1 bis 4, bei dem in den phenolhaltigen, wässrigen Lösungen zusätzlich noch bis zu 5 Gew.% Aceton enthalten sind.

6. Verfahren nach Anspruch 5, bei dem die phenolhaltigen, wässrigen Lösungen vor der Extraktion einer Destillation unterworfen werden, wobei zumindest eine Teilmenge des Acetons abgetrennt wird.

7. Verfahren nach einem der Ansprüche 1 bis 6, bei dem der Gehalt an Methylisobutylketon in den wässrigen Lösungen nach der Extraktion durch Destillation verringert wird.

8. Verfahren nach einem der Ansprüche 1 bis 7, bei dem Phenol und gegebenenfalls andere organische Komponenten durch Destillation aus dem aus der Extraktion erhaltenen Methylisobutylketon-haltigen Gemisch zurückgewonnen werden.

9. Verfahren nach einem der Ansprüche 1 bis 8, bei dem der Gehalt an organischen Komponenten in den wässrigen Lösungen nach der Extraktion zusätzlich durch Überleiten über Aktivkohle verringert wird.

10. Verfahren nach einem der Ansprüche 1 bis 9, bei dem die wässrigen Lösungen vor der Extraktion auf pH-Werte <7 angesäuert werden.

## Claims

1. A process for extracting phenol-containing aqueous solutions in which a mixture containing 60 to 99 wt.% of methyl isobutyl ketone, 1 to 40 wt.% of anisole and 0 to 20 wt.% of mesitylene, with respect to the mixture, is used as extraction agent.

2. A process according to Claim 1, in which the phenol concentration in the phenol-containing aqueous solutions to be extracted is 1-15 wt.%.

3. A process according to one of Claims 1 or 2, in which the phenol-containing aqueous solutions arise entirely or partly from the production of bisphenol A.

4. A process according to one of Claims 1 to 3, in which the phenol-containing aqueous solutions contain sodium sulfate.

5. A process according to one of Claims 1 to 4, in which in addition up to 5 wt.% of acetone is also present in the phenol-containing aqueous solutions.

6. A process according to Claim 5, in which the phenol-containing aqueous solutions are subjected to a distillation process prior to extraction, wherein at least some of the acetone is separated.

7. A process according to one of Claims 1 to 6, in which the concentration of methyl isobutyl ketone in the aqueous solutions after extraction is reduced by distillation.

8. A process according to one of Claims 1 to 7, in which phenol and optionally other organic components are recovered by distillation from the methyl isobutyl ketone-containing mixture obtained from the extraction process.

9. A process according to one of Claims 1 to 8, in which the concentration of organic components in the aqueous solutions after extraction is also reduced by passage over activated carbon.

10. A process according to one of Claims 1 to 9, in which the aqueous solutions are acidified to a pH <7 prior to extraction.

## Revendications

1. Procédé d'extraction de solutions aqueuses contenant du phénol, dans lequel comme agent d'extraction, on met en oeuvre un mélange contenant 60 à 99% en poids de méthylisobutylcétone, 1 à 40% en poids d'anisole et 0 à 20% en poids de mésitylène, sur base du mélange.

2. Procédé selon la revendication 1, dans lequel la teneur en phénol dans les solutions aqueuses contenant du phénol à extraire, se situe dans l'intervalle allant de 1 à 15% en poids.

3. Procédé selon la revendication 1 ou 2, dans lequel les solutions aqueuses contenant du phénol dérivent complètement ou partiellement de la production de bisphénol A.

4. Procédé selon l'une des revendications 1 à 3, dans lequel les solutions aqueuses contenant du phénol contiennent du sulfate de sodium.

5. Procédé selon l'une des revendications 1 à 4, dans lequel les solutions aqueuses contenant du phénol contiennent en outre, encore jusqu'à 5% en poids d'acétone.

6. Procédé selon la revendication 5, dans lequel les solutions aqueuses contenant du phénol sont soumises avant l'extraction, à une distillation où au moins une quantité partielle de l'acétone est séparée.

7. Procédé selon l'une des revendications 1 à 6, dans lequel la teneur en méthylisobutylcétone dans les solutions aqueuses après l'extraction est réduite par distillation.

8. Procédé selon l'une des revendications 1 à 7, dans lequel le phénol et le cas échéant, d'autres composants organiques sont récupérés par distillation du mélange contenant la méthylisobutylcétone, obtenu de l'extraction.

9. Procédé selon l'une des revendications 1 à 8, dans lequel la teneur en composants organiques dans les solutions aqueuses après l'extraction est encore réduite par passage sur charbon actif.

10. Procédé selon l'une des revendications 1 à 9, dans lequel les solutions aqueuses sont acidifiées à pH < 7 avant l'extraction.
